# EUROPÄISCHE PATENTSCHRIFT

(11) **EP 0 657 413 B1**
(45) Veröffentlichungstag und Bekanntmachung des Hinweises auf die Patenterteilung: **22.10.1997**
(21) Anmeldenummer: 94118673.6
(22) Anmeldetag: 28.11.1994
(51) Int. Cl.: C07C 51/377, C07C 67/317, C07C 53/18, C07C 53/21, C07C 69/63

(54) **Verfahren zur Herstellung von aliphatischen Omega-Difluorcarboxylverbindungen**
Process for preparing aliphatic omega-difluorocarboxylic compounds
Procédé de préparation de composés omega-difluorocarboxyliques aliphatiques

(30) Priorität: 10.12.1993 DE 4342187
(43) Veröffentlichungstag der Anmeldung: 14.06.1995
(73) Patentinhaber: BAYER AG, 51368 Leverkusen (DE)
(72) Erfinder: Bielefeldt, Dietmar Dr., D-40883 Ratingen (DE)

(56) Entgegenhaltungen:
- DE-C- 910 778
- COLLECTION OF CZECHOSLOVAK CHEMICAL COMMUNICATIONS, Bd.35, Nr.4, April 1970, PRAGUE CS Seiten 1302 - 1306 O.PALETA ET AL. 'PREPARATION OF FLUOROACETIC ACIDS BASED ON TRIFLUOROCHLOROETHYLENE.'

## Beschreibung

Die vorliegende Erfindung betrifft ein wirtschaftliches Herstellungsverfahren für aliphatische ω-Difluorcarboxylverbindungen aus den entsprechenden ω-Monohalogen-difluor-carboxylverbindungen.

Difluoressigsäure ist ein Zwischenprodukt zur Herstellung von Pflanzenschutzmitteln. Beispielsweise wird sie benötigt für die Herstellung von 5-Difluormethyl-1,3,4-thiazol-2-yloxyderivaten (siehe DE-A1 38 21 597). Längerkettige fluorierte Alkancarbonsäuren und deren Salze sind als oberflächenaktive Substanzen bekannt und können als Emulgatoren bei der Polymerisation von fluoriertem Monomeren eingesetzt werden (siehe Ullmann's Encyclopedia of Industrial Chemistry, Vol. A11, S. 371 ff (1988)).

Difluoressigsäure läßt sich aus Folgeprodukten des Tetrafluorethens oder durch Oxidation von 3,3'-Difluorpropenen mit Kaliumpermanganat synthetisieren (siehe z.B. J. Amer. Chem. Soc. 74, 1426 (1952) und J. Amer. Chem. Soc. 71, 343 (1949) und die dort zitierte Literatur. Beide Herstellungsmethoden sind wirtschaftlich wenig interessant. Insbesondere sind beim erstgenannten Verfahren die Ausgangsprodukte nur schwierig zugänglich und beim letztgenannten Verfahren ergibt sich das Problem der Entsorgung der entstehenden Manganhydroxide und -oxide.

Es wurde nun ein Verfahren zur Herstellung von ω-Difluorcarboxylverbindungen der Formel (I)

CF₂H―(CH₂)ₙ―CO₂R (I),

in der
- n: für Null oder eine ganze Zahl von 1 bis 5 und
- R: für Wasserstoff C₁-C₁₀-Alkyl, C₅-C₇-Cycloalkyl, C₆-C₁₀-Aryl oder C₇-C₁₂-Aralkyl
stehen, gefunden, das dadurch gekennzeichnet ist, daß man eine Difluorcarboxylverbindung der Formel (II)

CF₂X―(CH₂)ₙ―CO₂R (II),

in der
- X: für Chlor, Brom oder Iod steht und
- n und R: die bei Formel (I) angegebene Bedeutung haben,
mit Wasserstoff in Gegenwart eines auf einem inerten Trägermaterial aufgebrachten Katalysators, der eines oder mehrere Metalle der VIII. Gruppe des Periodensystems enthält, bei 120 bis 250°C in Kontakt bringt.

In Formel (II) stehen X vorzugsweise für Chlor, n vorzugsweise für Null, 1 oder 2 und R vorzugsweise für Wasserstoff oder C₁-C₄-Alkyl. Dementsprechend werden mit dem erfindungsgemäßen Verfahren vorzugsweise Difluorcarboxylverbindungen der Formel (I) hergestellt, bei denen n für Null, 1 oder 2 und R für Wasserstoff oder C₁-C₄-Alkyl steht. Ganz besonders bevorzugt stellt man erfindungsgemäß Difluoressigsäure aus Monochlor-difluor-essigsäure her.

Die Ausgangsprodukte der Formel (II) sind im Handel erhältlich oder auf einfache Weise und im Prinzip bekannte Weise herstellbar, beispielsweise aus Tetrachlorkohlenstoff, einem Olefin und Kohlenmonoxid (siehe J. org. Chem. 35, 2982 (1970)) und anschließender Fluorierung mit HF (siehe M. Hudlichy, Chemistry of Organic Fluorine Compounds, New York (1972)).

Als Wasserstoff kann man handelsüblichen Wasserstoff einsetzen. Bezogen auf 1 g-Atom X, das in der eingesetzten Verbindung der Formel (II) enthalten ist, kann man beispielsweise 1 bis 100 Mol Wasserstoff einsetzen. Vorzugsweise liegt diese Menge im Bereich 2 bis 50 Mol.

Die in das erfindungsgemäße Verfahren einzusetzenden Katalysatoren können als Metalle beispielsweise Palladium, Platin und/oder Nickel enthalten. Die Metalle können als solche oder in Form von Verbindungen vorliegen. Palladium in elementarer Form ist bevorzugt. Bevorzugte Trägermaterialien sind Aluminiumoxid und Lithium-Aluminium-Spinell. Gerechnet als Metall können die Katalysatoren z.B. 0,01 bis 10 Gew.-% Metall enthalten. Vorzugsweise liegt diese Menge bei 0,1 bis 2 Gew.-%. Die Katalysatoren können gegebenenfalls mit weiteren Haupt- und/oder Nebengruppenmetallen und/oder Verbindungen von Haupt- und/oder Nebengruppenmetallen dotiert sein. Beispiele für solche Dotierungen sind Salze von Blei, Silber, Indium, Vanadium und Thallium. Die Dotierungen können beispielsweise in Mengen von 0,1 bis 100 Gew.-%, bezogen auf das bzw. die Metalle der VIII. Gruppe des Periodensystems vorliegen.

Das erfindungsgemäße Verfahren kann bei den verschiedensten Drucken durchgeführt werden, beispielsweise bei 0,1 bis 16 bar. Bevorzugt sind Drucke im Bereich 1 bis 5 bar, besonders bevorzugt ist Normaldruck. Die Reaktionstemperatur liegt vorzugsweise im Bereich 150-200°C. Druck und Temperatur werden vorzugsweise so aufeinander abgestimmt, daß die jeweils eingesetzte Verbindung der Formel (II) gasförmig mit dem Katalysator in Kontakt kommt.

Pro Stunde kann man beispielsweise 10 bis 2 000 g einer Verbindung der Formel (II) über einen Liter des Katalysators leiten. Vorzugsweise beträgt die Katalysatorbelastung 50 bis 500 g/l x h. Man kann auch in Gegenwart von Inertgasen arbeiten.

Das den Reaktionsraum verlassende Gemisch kann man beispielsweise so aufarbeiten, daß man zunächst die kondensierbaren Anteile kondensiert und die hergestellte Verbindung der Formel (I) isoliert, indem man das Kondensat fraktioniert destilliert.

Unumgesetztes Ausgangsprodukt und überschüssigen Wasserstoff kann man erneut in das erfindungsgemäße Verfahren einsetzen.

Das erfindungsgemäße Verfahren hat eine Reihe von Vorteilen. Man kann auf einfache und wirtschaftliche Weise ein ziemlich reines Produkt erhalten. Die erzielbaren Umsätze und/oder Selektivitäten sind hoch. Im wesentlichen entsteht ein gasförmiges Nebenprodukt (Halogenwasserstoff), das auf einfache Weise abtrennbar ist.

Es ist überraschend, daß mit dem erfindungsgemäßen Verfahren aus einer Monohalogen-difluor-Ausgangsverbindung selektiv das Halogen abgespalten werden kann, ohne daß eine nennenswerte Abspaltung von Fluoratomen erfolgt.

### Beispiele

### Allgemeine Arbeitsvorschrift:

Ein senkrecht stehendes, beheizbares Quarzrohr mit Wasserstoffzuleitung wurde mit 200 ml des jeweils angegebenen Katalysators gefüllt. Das Zudosieren von Monochlor-difluor-essigsäure erfolgte über eine Dosierpumpe, das Zudosieren von Wasserstoff mittels eines Rotameters. Nach Spülung und Trocknung des Katalysators mit Stickstoff bei Normaldruck wurde die Monochlor-difluor-essigsäure und der Wasserstoff (beides gasförmig) in den jeweils angegebenen Mengen bei der jeweils angegebenen Temperatur durch das Quarzrohr geleitet. Das das Quarzrohr verlassende Reaktionsgemisch wurde bei -78°C kondensiert und anschließend destilliert. So wurden mindestens zu 98 % reine Produkte erhalten, die ohne weitere Reinigung weiter umgesetzt werden können.

### Eingesetzte Katalysatoren:

A 5 g Palladium pro Liter Aluminiumoxid, dotiert mit 0,25 Gew.-% Silbernitrat (bezogen auf Palladium).
B 5 g Palladium pro Liter Aluminiumoxid, dotiert mit 0,25 Gew.-% Thallium(I)-nitrat (bezogen auf Palladium).
C 5 g Palladium pro Liter Aluminiumoxid.
D 18 g Palladium pro Liter Lithium-Aluminium-Spinell.
E wie D, jedoch dotiert mit 60 Gew.-% Vanadiumoxid und 30,5 Gew.-% Bleidiacetat (jeweils bezogen auf Palladium).

Einzelheiten der durchgeführten Beispiele sind aus der folgenden Tabelle ersichtlich.

| Beispiel Nr. | Katalysator | Temperatur [°C] | Belastung [g/l x h] | Molverhältnis H₂:Edukt | Umsatz [%] | Selektivität [%] |
|---|---|---|---|---|---|---|
| 1 | A | 150 | 130 | 22,3 | 33 | 79 |
| 2 | A | 150 | 130 | 11,1 | 9,6 | 86 |
| 3 | B | 200 | 130 | 22,3 | 39 | 86 |
| 4 | C | 150 | 130 | 22,3 | 43 | 91 |
| 5 | C | 250 | 130 | 22,3 | 99 | 32 |
| 6 | D | 200 | 25 | 65 | 80 | 83 |
| 7 | D | 150 | 25 | 65 | 61 | 78 |
| 8 | D | 150 | 91 | 12,7 | 64 | 73 |
| 9 | D | 150 | 79 | 14,8 | 51 | 90 |
| 10 | E | 150 | 136 | 15 | 95 | 95 |
| 11 | E | 175 | 136 | 14,9 | 99 | 99 |

## Patentansprüche

1. Verfahren zur Herstellung von ω-Difluorcarboxylverbindungen der Formel (I)
CF₂H―(CH₂)ₙ―CO₂R (I),
in der
n für Null oder eine ganze Zahl von 1 bis 5 und
R für Wasserstoff C₁-C₁₀-Alkyl, C₅-C₇-Cycloalkyl, C₆-C₁₀-Aryl oder C₇-C₁₂-Aralkyl
stehen,
dadurch gekennzeichnet, daß man eine Difluorcarboxylverbindung der Formel (II)
CF₂X―(CH₂)ₙ―CO₂R (II),
in der
X für Chlor, Brom oder Iod steht und
n und R die bei Formel (I) angegebene Bedeutung haben,
mit Wasserstoff in Gegenwart eines auf einem inerten Trägermaterial aufgebrachten Katalysators, der eines oder mehrere Metalle der VIII. Gruppe des Periodensystems enthält, bei 120 bis 250°C in Kontakt bringt.

2. Verfahren nach Anspruch 1, dadurch gekennzeichnet, daß in den Formeln (I) und (II) n für Null, 1 oder 2 und R für Wasserstoff oder C₁-C₄-Alkyl stehen und in Formel (II) X für Chlor steht.

3. Verfahren nach Ansprüchen 1 und 2, dadurch gekennzeichnet, daß man, bezogen auf 1 g-Atom X, das in der eingesetzten Verbindung der Formel (II) enthalten ist, 1 bis 100 Mol Wasserstoff einsetzt.

4. Verfahren nach Ansprüchen 1 bis 3, dadurch gekennzeichnet, daß der Katalysator Palladium, Platin und/oder Nickel in metallischer Form oder in Form von Verbindungen und als Trägermaterial Aluminiumoxid oder Lithium-Aluminium-Spinell enthält.

5. Verfahren nach Ansprüchen 1 bis 4, dadurch gekennzeichnet, daß der Katalysator 0,01 bis 10 Gew.-% Metall enthält.

6. Verfahren nach Ansprüchen 1 bis 5, dadurch gekennzeichnet, daß der Katalysator zusätzlich mit weiteren Haupt- und/oder Nebengruppenmetallen und/oder Verbindungen von Haupt- und/oder Nebengruppenmetallen dotiert ist.

7. Verfahren nach Ansprüchen 1 bis 6, dadurch gekennzeichnet, daß man es bei 150 bis 200°C und Drucken im Bereich 0,1 bis 16 bar durchführt.

8. Verfahren nach Ansprüchen 1 bis 7, dadurch gekennzeichnet, daß man Druck und Temperatur so aufeinander abstimmt, daß die jeweils eingesetzte Verbindung der Formel (II) gasförmig mit dem Katalysator in Kontakt kommt.

9. Verfahren nach Ansprüchen 1 bis 8, dadurch gekennzeichnet, daß man pro Stunde 10 bis 2 000 g einer Verbindung der Formel (II) über einen Liter des Katalysators leitet.

10. Verfahren nach Ansprüchen 1 bis 9, dadurch gekennzeichnet, daß man das den Reaktionsraum verlassende Gemisch aufarbeitet, indem man die kondensierbaren Anteile kondensiert und die hergestellte Verbindung der Formel (I) isoliert, indem man das Kondensat fraktioniert destilliert.

## Claims

1. Process for the preparation of ω-difluorocarboxyl compounds of the formula (I)
CF₂H―(CH₂)ₙ―CO₂R (I),
in which
n represents zero or an integer from 1 to 5 and
R represents hydrogen, C₁-C₁₀-alkyl, C₅-C₇-cycloalkyl, C₆-C₁₀-aryl or C₇-C₁₂-aralkyl,
characterized in that a difluorocarboxyl compound of the formula (II)
CF₂X―(CH₂)ₙ―CO₂R (II),
in which
X represents chlorine, bromine or iodine and
n and R have the meaning given in the case of formula (I),
is brought into contact with hydrogen in the presence of a catalyst which is applied to an inert support material and comprises one or more metals of group VIII of the Periodic Table at 120 to 250°C.

2. Process according to Claim 1, characterized in that, in the formulae (I) and (II), n represents zero, 1 or 2 and R represents hydrogen or C₁-C₄-alkyl, and in formula (II), X represents chlorine.

3. Process according to Claims 1 and 2, characterized in that 1 to 100 mol of hydrogen are employed per g atom of X contained in the compound of the formula (II) employed.

4. Process according to Claims 1 to 3, characterized in that the catalyst comprises palladium, platinum and/or nickel in metallic form or in the form of compounds and, as the support material, aluminium oxide or lithium aluminium spinel.

5. Process according to Claims 1 to 4, characterized in that the catalyst comprises 0.01 to 10 % by weight of metal.

6. Process according to Claims 1 to 5, characterized in that the catalyst is additionally doped with other main group and/or sub-group metals and/or compounds of main group and/or sub-group metals.

7. Process according to Claims 1 to 6, characterized in that it is carried out at 150 to 200°C under pressures in the range from 0.1 to 16 bar.

8. Process according to Claims 1 to 7, characterized in that the pressure and temperature are coordinated with one another such that the particular compound of the formula (II) employed comes into contact with the catalyst in gaseous form.

9. Process according to Claims 1 to 8, characterized in that 10 to 2000 g per hour of a compound of the formula (II) are passed over one litre of the catalyst.

10. Process according to Claims 1 to 9, characterized in that the mixture leaving the reaction space is worked up by condensing the condensable portions isolating the compound of the formula (I) prepared by subjecting the condensate to fractional distillation.

## Revendications

1. Procédé de préparation de composés ω-difluorocarboxyliques de formule (I)
CF₂H - (CH₂)ₙ - CO₂R (I)
dans laquelle
n est égal à 0 ou à un nombre entier de 1 à 5, et
R représente un atome d'hydrogène ou un reste alkyle en C₁-C₁₀, cycloalkyle en C₅-C₇, aryle en C₆-C₁₀ ou aralkyle en C₇-C₁₂,
caractérisé en ce que l'on met en contact, à une température de 120 à 250°C, un composé difluorocarboxylique de formule (II)
CF₂X - (CH₂)ₙ - CO₂R (II)
dans laquelle
X représente le chlore, le brome ou l'iode et
n et R ont la signification indiquée dans la formule (I),
avec de l'hydrogène en présence d'un catalyseur contenant un ou plusieurs métaux du groupe VIII du système périodique, déposé sur un support inerte.

2. Procédé selon la revendication 1, caractérisé en ce que, dans les formules (I) et (II), n est 0, 1 ou 2 et R est un atome d'hydrogène ou un reste alkyle en C₁-C₄, et, dans la formule (II), X représente le chlore.

3. Procédé selon les revendications 1 et 2, caractérisé en ce que l'on utilise 1 à 100 mol d'hydrogène pour 1 atome-gramme de X contenu dans le composé de formule (II) utilisé.

4. Procédé selon les revendications 1 à 3, caractérisé en ce que le catalyseur contient du palladium, du platine et/ou du nickel sous forme métallique ou sous forme de composés et, comme support, de l'oxyde d'aluminium ou une spinelle de lithium et d'aluminium.

5. Procédé selon les revendications 1 à 4, caractérisé en ce que le catalyseur contient 0,01 à 10 % en masse de métal.

6. Procédé selon les revendications 1 à 5, caractérisé en ce que le catalyseur est en outre dopé avec d'autres métaux des groupes principaux et/ou secondaires et/ou des composés de métaux des groupes principaux et/ou secondaires.

7. Procédé selon les revendications 1 à 6, caractérisé en ce qu'il s'effectue à une température de 150 à 200°C et sous des pressions comprises entre 0,1 et 16 bars.

8. Procédé selon les revendications 1 à 7, caractérisé en ce que l'on règle la pression et la température l'une par rapport à l'autre de façon que le composé de formule (II) utilisé vienne sous forme gazeuse en contact avec le catalyseur.

9. Procédé selon les revendications 1 à 8, caractérisé en ce que l'on fait passer 10 à 2000 g d'un composé de formule (II) sur un litre de catalyseur par heure.

10. Procédé selon les revendications 1 à 9, caractérisé en ce que l'on traite le mélange sortant de l'espace réactionnel en condensant les constituants condensables et en isolant le composé préparé de formule (I) par distillation fractionnée du condensat.
